# EUROPÄISCHE PATENTSCHRIFT

(11) **EP 3 059 222 B1**
(45) Veröffentlichungstag und Bekanntmachung des Hinweises auf die Patenterteilung: **27.06.2018**
(21) Anmeldenummer: 15155563.8
(22) Anmeldetag: 18.02.2015
(51) Int. Cl.: C07C 67/03, C07C 69/82, C08K 5/12

(54) **Verfahren zur Herstellung von Estergemischen durch Umesterung**
Process for the preparation of ester mixtures by transesterification
Procédé pour la préparation des mélanges d'esters par transestérification

(43) Veröffentlichungstag der Anmeldung: 24.08.2016
(73) Patentinhaber: Evonik Degussa GmbH, 45128 Essen (DE)
(72) Erfinder: Boeck, Florian Sebastian, 48143 Münster (DE); Grass, Michael, 45721 Haltern am See (DE); Woldt, Benjamin, 44892 Bochum (DE)

(56) Entgegenhaltungen:
- WO-A1-2009/095126
- US-A1- 2014 336 320

## Beschreibung

Die vorliegende Erfindung betrifft ein Verfahren zur Herstellung eines Estergemisches.

Die Verwendung von Estern der Terephthalsäure, der Phthalsäure, der Cyclohexandicarbonsäuren, der Adipinsäure, der Bernsteinsäure, der Citronensäure und anderer organischer Säuren als Weichmacher für Polymere ist seit längerem bekannt. Diese Ester werden hauptsächlich durch Veresterung der Säuren oder deren Derivate mit Alkoholen hergestellt.

Je nach der eingesetzten Säure beziehungsweise des eingesetzten Säurederivates und dem gewählten Alkohol variieren die Eigenschaften der resultierenden (ungemischten) Ester deutlich. Es resultieren Ester, welche als Weichmacher für verschiedenste Anwendungen geeignet sind, oftmals neben einer oder zwei sehr guten Eigenschaften jedoch auch ungünstige Eigenschaften aufweisen, welche dann den Einsatz des jeweiligen Esters stark beschränken können. Beispielsweise könnten Dioctylphthalat, Di*iso*decylphthalat, Di*iso*nonylphthalat, Trioctyltrimellitat und Tri*iso*nonyltrimellitat gut in den unterschiedlichen Hochtemperaturanwendungen eingesetzt werden, da sie eine hohe Wärmebeständigkeit und geringe Flüchtigkeit aufweisen. Der Einsatz im Bereich dieser Anwendungen unterliege gemäß dem Dokument US 2014/0096703 A1 jedoch Beschränkungen, da diese Ester eine geringe Verträglichkeit mit den für diese Anwendungen eingesetzten Polymeren aufwiesen. Die Dibutyl-Derivate dieser Ester wiesen eine hohe Verträglichkeit mit Polymeren, beispielsweise PVC auf, kämen jedoch für Hochtemperaturanwendungen aufgrund ihrer geringen Wärmebeständigkeit nicht in Betracht. Um dennoch Weichmachersysteme bereitstellen zu können, welche eine gute Wärmebeständigkeit bei gleichzeitig guter Verträglichkeit aufweisen, schlägt das Dokument US 2014/0096703 A1 vor, ein Estergemisch aus C₈- und C₁₀-Estern der Terephthalsäure einzusetzen, welches neben den ungemischten Estern auch den Mischester, welcher einen C₈- und einen C₁₀-Alkoholrest aufweist, enthält.

Das Dokument KR 2013/0035493 A legt das Problem dar, dass Dibutylterephthalat zwar eine hohe Geschwindigkeit bei dem Eindringen in das Harz und beim Schmelzen aufweise, jedoch einen unerwünscht hohen Migrationsverlust zeige, während bei Diethylhexylterephthalat kaum ein Migrationsverlust auftrete, das Eindringen in das Harz und das Schmelzen aber unakzeptabel lange dauere. Zur Verbesserung schlägt auch dieses Dokument vor, Estergemische umfassend den Mischester, welcher einen C₄- und einen C₈-Alkoholrest enthält, einzusetzen.

Das Dokument WO 2008/140177 A1 schlägt vor, Estergemische aus C₈- und C₉-Estern der Terephthalsäure herzustellen, welche wiederum auch den Mischester enthalten, und beschreibt, dass eine Verbesserung der Verarbeitbarkeit der Weichmacher-Zubereitungen durch die Variation der Verhältnisse der einzelnen Ester im Estergemisch erreicht werden könne.

Bekannt sind auch Estergemische umfassend Mischester hergestellt aus Cyclohexandicarbonsäuren mit unterschiedlichen Alkoholen (WO 2011/115757 A1).

Auch Gemische von Zitronensäureestern, welche neben den entsprechenden ungemischten Estern auch Mischester mit C₅- und C₉-Alkoholresten enthalten, sind bekannt (US 8,431,638 B2).

Diese Estermischungen werden im Stand der Technik - unabhängig von ihrem Grundgerüst - durch das Verestern der entsprechenden Säuren oder Säurederivate mit einer Alkoholmischung bereitgestellt, welche die Alkoholreste der ungemischten Ester und des Mischesters beziehungsweise der Mischester liefert. So offenbart das Dokument US 2014/0336320 A1 zahlreiche Beispiele, in welchen mittels unterschiedlichster Verfahrensführung Mischester hergestellt werden.

Wie beispielsweise in dem Dokument WO 2008/140177 A1 gezeigt wird, ist es jedoch nicht möglich, mittels der Zusammensetzung des Alkoholgemisches die Verteilung der resultierenden Ester gezielt einzustellen. So enthalten die in den Beispielen dieses Dokumentes hergestellten Estergemische die C₈- und C₉-Alkoholreste nicht in jenem Mengenverhältnis, in welchem diese Reste im eingesetzten Alkoholgemisch enthalten sind und wohl auch in das System eingeführt werden sollten. Auch bilden sich die C₈/C₈-Ester, die C₈/C₉-Ester und die C₉/C₉-Ester nicht in dem aufgrund von statistischen Überlegungen erwarteten Molverhältnissen, sondern in deutlich davon abweichenden Verhältnissen. Im Beispiel 1 des Dokument WO 2008/140177 A1 wird bei einem statistischen Erwartungswert von 25:50:25 (Annahme: äquimolarer Einbau der Alkoholreste) ein Molverhältnis der Ester von 10:54:36 erhalten, was einer nicht steuerbaren Abweichung um (|25-10| + |50-54| + |25-36| =) 30 Punkten entspricht (die Berechnung der Abweichung in Punkten wird später im Text erläutert). Während in diesem Beispiel die beiden Alkohole zwar in einem Molverhältnis von 1:1 eingesetzt werden, enthält das resultierende Estergemisch die beiden Alkoholreste in einem Molverhältnis von 37:63. Das Dokument schreibt diese Abweichungen vom statistischen Erwartungswert den unterschiedlichen Reaktionsgeschwindigkeiten der einzelnen Veresterungsreaktionen zu.

Dieses Problem der "Nichtsteuerbarkeit" der Estergemisch-Zusammensetzungen bei der Herstellung durch Veresterung der Terephthalsäure wird im Dokument KR 2013/0035493 A gelöst, indem die Ester des Gemisches getrennt hergestellt und im gewünschten Verhältnis abgemischt werden. Das Dokument enthält jedoch keine Offenbarung dazu, wie ein Mischester separat hergestellt werden kann, ohne dass sich gleichzeitig die beiden ungemischten Diester bilden. Dies scheint mit kommerziell vertretbarem Aufwand auch gar nicht möglich. Die Bereitstellung des puren Mischesters müsste durch dessen Abtrennung von den ungemischten Estern während eines Trennungsschrittes erfolgen. Soll dann dieser aufgereinigte Mischester mit den beiden ungemischten Estern gemäß Offenbarung des Dokumentes KR 2013/0035493 A abgemischt werden, müssen für die Herstellung eines Estergemisches beispielsweise zumindest drei Veresterungsreaktionen und eine anschließende Abmischung durchgeführt werden, was apparativ und zeitlich aufwändig ist.

Einen anderen Weg beschreibt das Dokument US 8,431,638 B2 für die Herstellung von gemischten Zitronensäureestern. Gemische umfassend diese Ester können hergestellt werden, indem Zitronensäurepentylester mit Zitronensäurenonylestern umgeestert werden. Diese Vorgehensweise erfordert dennoch drei getrennte Verfahrensschritte: zwei Veresterungen und eine Umesterung.

Der vorliegenden Erfindung lag nun die Aufgabe zugrunde, einige, vorzugsweise alle der oben genannten Nachteile des Standes der Technik zu überwinden. Mit Vorzug sollte ein Verfahren entwickelt werden, in welchem Gemische umfassend Mischester und ungemischte Ester mit vorbestimmter Mengenverteilung der Ester gezielt hergestellt werden können, wobei der apparative und zeitliche Aufwand so gering wie möglich gehalten werden soll.

Diese Aufgabe wird gelöst durch ein Verfahren zur Herstellung eines Estergemisches gemäß Anspruch 1.

Gegenstand der vorliegenden Erfindung ist deshalb ein Verfahren zur Herstellung eines Estergemisches umfassend die Ester A(COOR¹)(COOR¹)ₓ, A(COOR¹)(COOR²)ₓ, A(COOR²)(COOR¹)ₓ und A(COOR²)(COOR²)ₓ, in welchem der Ester A(COOR¹)(COOR¹)ₓ mit weniger als 1 Mol-Äquivalent, bezogen auf die Anzahl seiner Esterfunktionen, des Alkohols R²OH in Anwesenheit eines Katalysators umgesetzt und dabei zum Sieden erhitzt wird, wobei der Alkohol R¹OH einen niedrigeren Siedepunkt bei einem bestimmten Druck aufweist als der Alkohol R²OH bei demselben Druck und mehr als 90 Mol-% des sich im Laufe des Verfahrens bildenden R¹OH während des Verfahrens aus dem Reaktionsgefäß entfernt werden bevor der Katalysator zerstört wird und
- A: für einen aromatischen, alicyclischen oder aliphatischen Rest steht,
- x: 1 oder 2 ist und
- R¹ und R²: unabhängig voneinander für substituierte oder nichtsubstituierte Arylreste oder lineare oder verzweigte, substituierte oder nicht substituierte Alkylreste mit 3 bis 20 Kohlenstoffatomen stehen.

Für x = 1 sind die Ester A(COOR¹)(COOR²)ₓ und A(COOR²)(COOR¹)ₓ ein und dieselbe Verbindung.

In dem erfindungsgemäßen Verfahren wird der Ester A(COOR¹)(COOR¹)ₓ mit weniger Alkohol R²OH umgesetzt, als zum vollständigen Austausch sämtlicher Alkoholreste der Esterfunktionen innerhalb des Moleküls A(COOR¹)(COOR¹)ₓ notwendig wäre. Überraschenderweise wurde festgestellt, dass dann die Zusammensetzung des resultierenden Estergemisches umfassend die ungemischten Ester A(COOR¹)(COOR¹)ₓ und A(COOR²)(COOR²)ₓ sowie die Mischester A(COOR¹)(COOR²)ₓ und A(COOR²)(COOR¹)ₓ gezielt - im Rahmen der sich für den Fall des vollständigen Einbaus der Alkoholreste OR² ergebenden Statistik - eingestellt werden kann, wenn der Ester A(COOR¹)(COOR¹)ₓ mit dem Alkoholrest des niedrigersiedenden Alkohols R¹OH vorgelegt und der höhersiedende Alkohol R²OH zugesetzt wird. Eine Steuerung der Estergemisch-Zusammensetzung ist hingegen nicht möglich, wenn in einer Veresterung oder Umesterung ein Alkoholgemisch (R¹OH + R²OH) der einzuführenden Alkoholreste eingesetzt wird oder zu dem Ester A(COOR²)(COOR²)ₓ mit dem Alkoholrest des schwererflüchtigen Alkohols R²OH der leichterflüchtige Alkohol R¹OH zugesetzt wird.

So ist es mittels des erfindungsgemäßen Verfahrens möglich, Estergemische bereitzustellen, in denen die unterschiedlichen Alkoholreste in einer vorbestimmten Mengenverteilung enthalten sind und in denen zudem die Mengenverteilung der enthaltenen Ester gezielt - im Rahmen der oben genannten Statistik - gesteuert werden kann. Somit ist es möglich, Estergemische bereitzustellen, deren Zusammensetzung geringere Abweichungen von der sich aufgrund der Statistik ergebenden Verteilung der Ester aufweist, als Estergemische, welche nach im Stand der Technik beschriebenen, ungesteuerten Verfahren hergestellt werden.

Für x = 1 ergeben sich die in der Tabelle 1 dargestellten statistischen Erwartungswerte der Estergemisch-Zusammensetzung.

**Tabelle 1: statistische Erwartungswerte Estergemisch-Zusammensetzung für x = 1**

| Einsatz A(COOR¹)₂ [R¹ - Äquivalente] | Einsatz R²OH [R²-Äquivalente] | Erwartungsmenge A(COOR¹)₂ [Mol-%] | Erwartungsmenge A(COOR¹)(COOR²) + A(COOR²)(COOR¹) [Mol-%] | Erwartungsmenge A(COOR²)₂ [Mol-%] |
|---|---|---|---|---|
| 2 | 0,2 | 81 | 18 | 1 |
| 2 | 0,4 | 64 | 32 | 4 |
| 2 | 0,6 | 49 | 42 | 9 |
| 2 | 0,8 | 36 | 48 | 16 |
| 2 | 1,0 | 25 | 50 | 25 |
| 2 | 1,2 | 16 | 48 | 36 |
| 2 | 1,4 | 9 | 42 | 49 |
| 2 | 1,6 | 4 | 32 | 64 |
| 2 | 1,8 | 1 | 18 | 81 |

Eine Quantifizierung der Abweichung der Estergemischzusammensetzung von der aus statistischen Überlegungen resultierenden Mengenverteilung der Ester im Estergemisch ist durch Summenbildung aller Beträge der Differenzen zwischen dem statistischem Erwartungswert, welcher sich ergibt, wenn man von einem vollständigen Einbau der Alkoholreste OR² ausgeht, und dem tatsächlichen Mol-Anteil jedes einzelnen Esters im Estergemisch für den Fall, dass sich die Summe der Mol-Anteile der oben genannten Ester im Estergemisch zu 100 addiert, möglich.

Mittels des erfindungsgemäßen Verfahrens gelingt es beispielsweise aus 5,5 Mol Di*iso*pentylterephthalat und 7,1 Mol *Iso*nonanol gezielt ein Estergemisch bereitzustellen, in dem die Molverteilung der enthaltenen Ester lediglich um 4 Punkte vom statistischen Erwartungswert abweicht und der Anteil der *Iso*pentylreste im Estergemisch bis auf 1 % dem anvisierten *Iso*pentylanteil entspricht.

In dem erfindungsgemäßen Verfahren kann der Rest A für aromatische, alicyclische oder aliphatische Reste stehen. In einer Ausführungsform steht A für einen aliphatischen Rest mit 4 bis 12 Kohlenstoffatomen, welcher 2 oder 3 Säurefunktionen umfasst und optional weitere funktionelle Gruppen trägt. In einer anderen Ausführungsform steht A für einen ungesättigten Alkylrest, welcher 4 bis 12 Kohlenstoffatome enthält, wobei er 2 oder 3 Säurefunktionen aufweist und optional weitere funktionelle Gruppen trägt. In einer weiteren Ausführungsform steht A für einen alicyclischen Rest, welcher 4 bis 8 Kohlenwasserstoffatome aufweist, an welche 2 oder 3 Carbonsäurefunktionen und optional weitere funktionale Reste angebunden sind. In einer weiteren Ausführungsform steht A für einen aromatischen Rest mit 2 oder 3 Carbonsäurefunktionen und optional weiteren funktionellen Gruppen.

Bevorzugt handelt es sich bei dem mittels des erfindungsgemäßen Verfahren hergestellten Estergemisch um ein Estergemisch der Phthalsäure, der Terephthalsäure, der Isophthalsäure, der 1,2-, 1,3- oder 1,4- Cyclohexandicarbonsäure, der Adipinsäure, der Sebacinsäure, der Maleinsäure, der Bernsteinsäure, der Furandicarbonsäure oder der Citronensäure.

Die Reste R¹ und R² können unabhängig voneinander ausgewählt werden aus substituierten oder nichtsubstituierten Arylresten, beispielsweise Benzylresten und linearen oder verzweigten, substituierten oder nicht substituierten Alkylresten mit 3 bis 20 Kohlenstoffatomen, solange der Alkohol R¹OH einen niedrigeren Siedepunkt bei einem bestimmten Druck aufweist als der Alkohol R²OH bei demselben Druck. In einer Ausführungsform sind die Reste R¹ und R² ausgewählt aus linearen oder verzweigten, substituierten oder nicht substituierten Alkylresten mit 3 bis 20 Kohlenstoffatomen, wobei vorzugsweise einer der Reste R¹ oder R² oder beide Reste R¹ und R² weitere funktionelle Gruppen, beispielsweise eine oder mehrere Mehrfachbindungen, Ether-, Aldehyd-, Keton-, Hydroxy- und/oder Halogenidgruppen enthält beziehungsweise enthalten. In einer anderen Ausführungsform sind die Reste R¹ und R² unabhängig voneinander ausgewählt aus Alkylresten, welche 3 bis 20, vorzugsweise 4 bis 15 und insbesondere 5 bis 11 Kohlenstoffatome enthalten, wobei die Alkylreste bevorzugt keine weiteren funktionellen Gruppen einschließlich Mehrfachbindungen aufweisen. Vorzugsweise sind R¹ und R² unabhängig voneinander ausgewählt aus Propyl-, Butyl-, *tert*-Butyl-, *Iso*butyl-, 2-Methylbutyl-, 3-Methylbutyl-, *n*-Pentyl-, *Iso*pentyl-, Hexyl-, Heptyl-, *Iso*heptyl-, Octyl-, *Iso*octyl-, 2-Ethylhexyl-, Nonyl-, *n*-Nonyl-, *Iso*nonyl-, Decyl-, *Iso*decyl-, 2-Propylheptyl-, Undecyl- und Tridecyl- Resten.

Mit Vorzug ist hierbei der Rest R¹ ausgewählt aus Propyl-, Butyl-, *tert*-Butyl-, *Iso*butyl-, 2-Methylbutyl-, 3-Methylbutyl-, *n*-Pentyl-, *Iso*pentyl-, Hexyl-, Heptyl- und *Iso*heptyl-Resten und gleichzeitig ist der Rest R² ausgewählt aus *n*-Pentyl-, *Iso*pentyl-, Hexyl-, Heptyl-, *Iso*heptyl-, Octyl-, *Iso*octyl-, 2-Ethylhexyl-, Nonyl-, *n*-Nonyl-, *Iso*nonyl-, Decyl-, *Iso*decyl-, 2-Propylheptyl-, Undecyl- und Tridecyl- Resten, jedoch mit der Maßgabe, dass der Alkohol R¹OH des Restes R¹ einen niedrigeren Siedepunkt bei einem bestimmten Druck aufweist als der Alkohol R²OH des Restes R² bei demselben Druck.

Es hat sich herausgestellt, dass das Verfahren mit besonders geringen Abweichungen von dem aufgrund der stöchiometrischen Einsatzmengen statistisch vorgestimmten Molverhältnis der gebildeten ungemischten Ester und Mischester durchgeführt werden kann, wenn die Siedepunkte der Alkohole R¹OH und R²OH sich um mindestens 10 °C, vorzugsweise um mindestens 25 °C und insbesondere um mindestens 40 °C unterscheiden. Bevorzugt weist der Alkohol R¹OH bei 1013 hPa einen um mindestens 10 °C, besonders bevorzugt einen um mindestens 20 °C, bevorzugt dazu einen um mindestens 30 °C, mit Vorzug einen um mindestens 40 °C, mit besonderem Vorzug einen um mindestens 50 °C und insbesondere einen um mindestens 60 °C niedrigeren Siedepunkt auf als der Alkohol R²OH bei demselben Druck.

Handelt es sich bei dem Alkohol R¹OH und/oder bei dem Alkohol R²OH um ein Isomerengemisch, so ist dies im Rahmen dieses Textes - wie in der Technischen Chemie üblich - durch das Präfix "*Iso*" gekennzeichnet. Isomerengemische weisen keinen scharfen Siedepunkt auf, sondern besitzen einen Siedebereich. Im Fall von Isomerengemischen wird die Differenz der Siedepunkte im Rahmen der vorliegenden Erfindung durch Differenzbildung zwischen der unteren Siedebereichsgrenze des höhersiedenden Alkohols und der oberen Siedebereichsgrenze des niedrigersiedenden Alkohols bestimmt.

Erfindungsgemäß wird in dem Verfahren weniger als 1 Mol-Äquivalent bezogen auf die Anzahl im Ester A(COOR¹)(COOR¹)ₓ enthaltener Esterfunktionen (entsprechend 1 Mol-Äquivalent R¹-Äquivalente) an R²OH eingesetzt. Bevorzugt wird der Ester A(COOR¹)(COOR¹)ₓ mit weniger als 0,98 Mol-Äquivalent, bevorzugt mit weniger als 0,95 Mol-Äquivalent und insbesondere mit weniger als 0,90 Mol-Äquivalent, bezogen auf die Anzahl seiner Esterfunktionen, des Alkohols R²OH umgesetzt. Es kann auch bevorzugt sein, den Ester A(COOR¹)(COOR¹)ₓ mit weniger als 0,85 Mol-Äquivalent, bevorzugt mit weniger als 0,80 Mol-Äquivalent und insbesondere mit weniger als 0,75 Mol-Äquivalent, bezogen auf die Anzahl seiner Esterfunktionen, des Alkohols R²OH umzusetzen. Auf diese Weise werden Estergemische erhalten, welche gegenüber dem Ester A(COOR²)(COOR²)ₓ deutlich günstigere, das heißt niedrigere, Geliertemperaturen aufweisen und sich gleichzeitig gegenüber A(COOR¹)(COOR¹)ₓ durch eine geringere und damit verbesserte Flüchtigkeit auszeichnen. Estergemische, welche besonders gut mit Polymeren, beispielsweise PVC, verarbeitbar sind, werden erhalten, wenn der Ester A(COOR¹)(COOR¹)ₓ mit weniger als 0,85 Mol-Äquivalent, bevorzugt mit weniger als 0,80 Mol-Äquivalent und insbesondere mit weniger als 0,75 Mol-Äquivalent, bezogen auf die Anzahl seiner Esterfunktionen, des Alkohols R²OH umgesetzt wird.

Mit Vorzug wird der Ester A(COOR¹)(COOR¹)ₓ mit mehr als 0,05 Mol-Äquivalent, bevorzugt mit mehr als 0,10 Mol-Äquivalent und insbesondere mit mehr als 0,20 Mol-Äquivalent, bezogen auf die Anzahl seiner Esterfunktionen, des Alkohols R²OH umgesetzt. In vielen Fällen ist es von Vorzug, den Ester A(COOR¹)(COOR¹)ₓ mit mehr als 0,25 Mol-Äquivalent, bevorzugt mit mehr als 0,30 Mol-Äquivalent und insbesondere mit mehr als 0,35 Mol-Äquivalent bezogen auf die Anzahl seiner Esterfunktionen, des Alkohols R²OH umzusetzen. Hierdurch können Estergemische erhalten werden, welche zur Herstellung von Estergemisch- und Polymer-haltigen Mitteln mit besonders guter Lagerfähigkeit geeignet sind. Eine weitere Verbesserung der Eigenschaften im Bereich der Flüchtigkeit wird möglich, wenn der Ester A(COOR¹)(COOR¹)ₓ mit mehr als 0,70 Mol-Äquivalent, bevorzugt mit mehr als 0,75 Mol-Äquivalent und insbesondere mit mehr als 0,80 Mol-Äquivalent, bezogen auf die Anzahl seiner Esterfunktionen, des Alkohols R²OH umgesetzt wird.

Ein bevorzugter Gegenstand der vorliegenden Erfindung ist ein Verfahren zur Herstellung eines Estergemisches umfassend die Ester Dipentylterephthalat, Di*iso*nonylterephthalat und Pentyl(*iso*nonyl)terephthalat, in welchem Dipentylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird.

Ein bevorzugter Gegenstand der vorliegenden Erfindung ist ein Verfahren zur Herstellung eines Estergemisches umfassend die Ester Dipentylterephthalat, Dipropylheptylterephthalat und Pentyl(propylheptyl)terephthalat, in welchem Dipentylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Propylheptanol umgesetzt und dabei zum Sieden erhitzt wird.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäuredi*iso*nonylester und Cyclohexandicarbonsäure-pentyl(*iso*nonyl)ester, in welchem Cyclohexandicarbonsäuredipentylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäuredipropylheptylester und Cyclohexandicarbonsäure-pentyl(propylheptyl)ester, in welchem Cyclohexandicarbonsäuredipentylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Propylheptanol umgesetzt und dabei zum Sieden erhitzt wird.

Ein bevorzugter Gegenstand der vorliegenden Erfindung ist ein Verfahren zur Herstellung eines Estergemisches umfassend die Ester Dipentylsuccinat, Di*iso*nonylsuccinat und Pentyl(*iso*nonyl)succinat, in welchem Dipentylsuccinat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird.

Ein bevorzugter Gegenstand der vorliegenden Erfindung ist ein Verfahren zur Herstellung eines Estergemisches umfassend die Ester Dipentylsuccinat, Dipropylheptylsuccinat und Pentyl(propylheptyl)succinat, in welchem Dipentylsuccinat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Propylheptanol umgesetzt und dabei zum Sieden erhitzt wird.

Ein bevorzugter Gegenstand der vorliegenden Erfindung ist ein Verfahren zur Herstellung eines Estergemisches umfassend die Ester Tripentylcitrat, Di*iso*nonyl(pentyl)citrat, Dipentyl(*iso*nonyl)citrat und Tri*iso*nonylcitrat, in welchem Tripentylcitrat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird.

Ein bevorzugter Gegenstand der vorliegenden Erfindung ist ein Verfahren zur Herstellung eines Estergemisches umfassend die Ester Tripentylcitrat, Dipropylheptyl(pentyl)citrat, Dipentyl(propylheptyl)citrat und Tripropylheptylcitrat, in welchem Tripentylcitrat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Propylheptanol umgesetzt und dabei zum Sieden erhitzt wird.

Vorzugsweise weist das in dem erfindungsgemäßen Verfahren, insbesondere das in diesen Ausführungsformen eingesetzte *Iso*nonanol einen durchschnittlichen Verzweigungsgrad von 1,0 bis 2,2 auf. Der durchschnittliche Verzweigungsgrad wird dabei bestimmt wie im Dokument US 2010/305255 A1 beschrieben. In einer anderen Ausführungsform enthält das in dem erfindungsgemäßen Verfahren, insbesondere das in obigen Ausführungsformen eingesetzte *Iso*nonanol weniger als 25 Mol-%, insbesondere weniger als 15 Mol-% Alkohole mit einer Kohlenstoffatomanzahl, welche kleiner oder größer ist als 9. Besonders bevorzugt weist das in dem erfindungsgemäßen Verfahren, insbesondere das in diesen Ausführungsformen eingesetzte *Iso*nonanol einen durchschnittlichen Verzweigungsgrad von 1,0 bis 2,2 auf und enthält gleichzeitig weniger als 25 Mol-%, insbesondere weniger als 15 Mol-% Alkohole mit einer Kohlenstoffatomanzahl, welche kleiner oder größer ist als 9 ist.

Unabhängig davon werden in dem erfindungsgemäßen Verfahren, insbesondere in den oben beschriebenen Ausführungsformen vorzugsweise Ester eingesetzt, welche *n*-Pentyl-, 2-Methylbutyl- und/oder 3-Methylbutyl-Reste enthalten. Eine bevorzugte Ausführungsform liegt vor, wenn in den oben beschriebenen Ausführungsformen die Pentyl-Reste Isomerengemische und somit *Iso*pentyl-Reste sind. Mit Vorzug liegt der Anteil der *n*-Pentyl-Reste basierend auf allen enthaltenen Pentyl-Resten bei mindestens 10 Mol-% oder 20 Mol-%, bevorzugt bei mindestens 30 Mol-%, besonders bevorzugt bei mindestens 40 Mol-%, ganz besonders bevorzugt bei mindestens 50 Mol-% und insbesondere bei mindestens 60 Mol-%, was mit dem Vorteil einer geringeren und damit für die Verarbeitung von Plastisolen günstigeren Viskosität verknüpft sein kann. In einer bevorzugten Ausführungsform liegt der der Anteil der *n*-Pentyl-Reste basierend auf allen enthaltenen Pentyl-Resten zwischen 10 und 90 Mol-%, bevorzugt bei 20 bis 80 Mol-% und insbesondere bei 30 bis 70 Mol-%.

Bei den Propylheptyl-Resten handelt es sich vorzugsweise um 2-Propylheptyl-Reste.

Ein bevorzugter Gegenstand der vorliegenden Erfindung ist ein Verfahren zur Herstellung eines Estergemisches umfassend die Ester Dibutylterephthalat, Dioctylterephthalat und Butyl(octyl)terephthalat, in welchem Dibutylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Octanol umgesetzt und dabei zum Sieden erhitzt wird. Bevorzugt handelt es sich hierbei bei den Octyl-Resten um Ethylhexyl-Reste, insbesondere um 2-Ethylhexyl-Reste und/oder bei den Butyl-Resten um *n*-Butyl-Reste.

Ein bevorzugter Gegenstand der vorliegenden Erfindung ist ein Verfahren zur Herstellung eines Estergemisches umfassend die Ester Dinonylterephthalat, Didecylterephthalat und Nonyl(decyl)terephthalat, in welchem Dinonylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Decanol umgesetzt und dabei zum Sieden erhitzt wird. Bevorzugt handelt es sich hierbei bei den Nonyl-Resten um *Iso*nonyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung eines Estergemisches umfassend die Ester Dioctylterephthalat, Didecylterephthalat und Octyl(decyl)terephthalat, in welchem Dioctylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Decanol umgesetzt und dabei zum Sieden erhitzt wird. Bevorzugt handelt es sich hierbei bei den Octyl-Resten um Ethylhexyl-Reste, insbesondere um 2-Ethylhexyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung eines Estergemisches umfassend die Ester Diheptylterephthalat, Dinonylterephthalat und Heptyl(nonyl)terephthalat, in welchem Diheptylterephthalat mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Nonanol umgesetzt und dabei zum Sieden erhitzt wird. Bevorzugt handelt es sich hierbei bei den Nonyl-Resten um *Iso*nonyl-Reste und/oder bei den Heptyl-Resten um *Iso*heptyl-Reste.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredibutylester, Cyclohexandicarbonsäuredioctylester und Cyclohexandicarbonsäurebutyl(octyl)ester, in welchem Cyclohexandicarbonsäuredibutylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Octanol umgesetzt und dabei zum Sieden erhitzt wird. Bevorzugt handelt es sich hierbei bei den Octyl-Resten um Ethylhexyl-Reste, insbesondere um 2-Ethylhexyl-Reste und/oder bei den Butyl-Resten um *n*-Butyl-Reste.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredinonylester, Cyclohexandicarbonsäuredidecylester und Cyclohexandicarbonsäurenonyl(decyl)ester, in welchem Cyclohexandicarbonsäuredinonylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Decanol umgesetzt und dabei zum Sieden erhitzt wird. Bevorzugt handelt es sich hierbei bei den Nonyl-Resten um *Iso*nonyl-Reste und/oder bei den Decyl-Resten um Propylheptyl-Reste, insbesondere um 2-Propylheptyl-Reste.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredioctylester, Cyclohexandicarbonsäuredidecylester und Cyclohexandicarbonsäureoctyl(decyl)ester, in welchem Cyclohexandicarbonsäuredioctylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Decanol umgesetzt und dabei zum Sieden erhitzt wird. Bevorzugt handelt es sich hierbei bei den Octyl-Resten um Ethylhexyl-Reste, insbesondere um 2-Ethylhexyl-Reste und/oder bei den Decyl-Resten um Propylheptyl-Reste, insbesondere um 2- Propylheptyl-Reste.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäurediheptylester, Cyclohexandicarbonsäuredinonylester und Cyclohexandicarbonsäureheptyl(nonyl)ester, in welchem Cyclohexandicarbonsäurediheptylester mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Nonanol umgesetzt und dabei zum Sieden erhitzt wird. Bevorzugt handelt es sich hierbei bei den Nonyl-Resten um *Iso*nonyl-Reste und/oder bei den Heptyl-Resten um *Iso*heptyl-Reste.

Besonders bevorzugt weist auch das in den voranstehenden Ausführungsformen des erfindungsgemäßen Verfahren eingesetzte *Iso*nonanol einen durchschnittlichen Verzweigungsgrad von 1,0 bis 2,2 auf und enthält vorzugsweise zudem gleichzeitig weniger als 25 Mol-%, insbesondere weniger als 15 Mol-% Alkohole mit einer Kohlenstoffatomanzahl, welche kleiner oder größer ist als 9 ist.

Zur Verbesserung der Steuerbarkeit der Zusammensetzung des aus der Umsetzung des Herstellungsverfahrens II resultierenden Estergemisches enthält die Reaktionsmischung während der Umsetzung mit Vorzug weniger als 0,5 Mol-Äquivalent, bevorzugt weniger als 0,1 Mol-Äquivalent, besonders bevorzugt weniger als 0,05 Mol-Äquivalent und insbesondere weniger als 0,01 Mol-Äquivalent an Alkoholen, welche nicht unter die Definition der Alkohole R¹OH und R²OH fallen, wobei sich die Mol-Äquivalente auf die Gesamtheit aller in der Reaktionsmischung enthaltenen Alkohole (entsprechend 1 Mol-Äquivalent) beziehen.

Die gewünschte Zusammensetzung des herzustellenden Estergemisches kann besonders gut gesteuert und das Verfahrensprodukt nach einem geringeren Aufarbeitungsaufwand direkt als Weichmacher oder Weichmacherkomponente genutzt werden, wenn die in dem erfindungsgemäßen Verfahren eingesetzten Komponenten umfassend A(COOR¹)(COOR¹)ₓ und R²OH weniger als 50 Vol.-%, vorzugsweise weniger als 35 Vol.-% und insbesondere weniger als 20 Vol.-%, weiter bevorzugt weniger als 10 Vol.-% an Komponenten enthält, welche keine Edukte, End- oder Zwischenprodukte der Umsetzung von A(COOR¹)(COOR¹)ₓ mit R²OH sind. Insbesondere sollten die eingesetzten Komponenten umfassend A(COOR¹)(COOR¹)ₓ und R²OH vorzugsweise insgesamt weniger als 15 Gew.-%, bevorzugt dazu weniger als 10 Gew.-% und insbesondere weniger als 5 Gew.-% an A(COOR¹)(COOH)ₓ und A(COOR²)(COOH)ₓ enthalten um die Steuerbarkeit der Umesterung nicht zu gefährden.

Mittels des erfindungsgemäßen Verfahrens werden Estergemische erhalten, welche bevorzugt mindestens 5 Mol-%, besonders bevorzugt mindestens 10 Mol-%, dazu bevorzugt mindestens 15 Mol-% und insbesondere mindestens 20 Mol-% des/der Mischester(s) (Mol-% der Ester A(COOR¹)(COOR²)ₓ und A(COOR²)(COOR¹)ₓ zusammen), basierend auf der Gesamtheit der Ester A(COOR¹)(COOR¹)ₓ, A(COOR¹)(COOR²)ₓ, A(COOR²)(COOR¹)ₓ und A(COOR²)(COOR²)ₓ enthalten. Bevorzugt kann es zudem sein, wenn die Gesamtheit der Ester A(COOR¹)(COOR¹)ₓ, A(COOR¹)(COOR²)ₓ, A(COOR²)(COOR¹)ₓ und A(COOR²)(COOR²)ₓ den/die Mischester in einer Menge von mindestens 25 Mol-%, mit Vorzug dazu mindestens 30 Mol-%, mit besonderem Vorzug mindestens 35 Mol-%, weiter bevorzugt mindestens 40 Mol-%, und insbesondere mindestens 45 Mol-% enthält.

Zudem sind Verfahren bevorzugt, deren Produkt maximal 50 Mol-%, bevorzugt maximal 45 Mol-% und insbesondere maximal 40 Mol-% des/der Mischester(s) (Mol-% der Ester A(COOR¹)(COOR²)ₓ und A(COOR²)(COOR¹)ₓ zusammen) enthält, basierend auf der Gesamtheit der Ester A(COOR¹)(COOR¹)ₓ, A(COOR¹)(COOR²)ₓ, A(COOR²)(COOR¹)ₓ und A(COOR²)(COOR²)ₓ.

In bevorzugten Ausführungsformen des erfindungsgemäßen Verfahrens ermöglicht dieses die Bereitstellung von Estergemischen, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Für x = 2 sind erfindungsgemäße Verfahren besonders bevorzugt, in denen Estergemische bereitgestellt werden, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ um weniger als 25 Punkte, vorzugsweise um weniger als 15 Punkte, insbesondere um weniger als 10 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Das erfindungsgemäße Verfahren wird vorzugsweise in Anwesenheit eines Katalysators oder mehrerer Katalysatoren, beispielsweise unter Verwendung von Brönstedt- oder Lewis-Säuren oder-Basen als Katalysator, durchgeführt. Als besonders geeignete Katalysatoren haben sich Schwefelsäure, Methansulfonsäure, *p*-Toluolsulfonsäure, Metalle oder deren Verbindungen erwiesen. Beispiele für besonders bevorzugte Metallkatalysatoren sind Zinnpulver, Zinn(II)oxid, Zinn(II)oxalat, Titansäureester wie Tetra*iso*propylorthotitanat oder Tetrabutylorthotitanat sowie Zirkoniumester wie Tetrabutylzirkonat sowie Natriummethanolat und Kaliummethanolat.

Das erfindungsgemäße Verfahren kann in dem Fachmann bekannten, typischen Veresterungsapparaturen unter üblichen Verfahrensbedingungen durchgeführt werden. Vorzugsweise findet das Verfahren bei Temperaturen bei oder oberhalb des Siedepunktes des Alkoholes R¹OH statt, so dass der bei der Reaktion gebildete Alkohol R¹OH bei dem vorgegebenen Druck aus dem Reaktionsgemisch abdestilliert werden kann. Zur Gewährleistung einer quantitativen Rückführung des in der Gasphase befindlichen Alkohols R²OH, sollte die Veresterungsapparatur vorzugsweise mit einer Kolonne ausgestattet sein. Unter dem Begriff "quantitativ" wird in diesem Zusammenhang zu mehr als 80 Mol-%, vorzugsweise zu mehr als 90 Mol-% und insbesondere zu mehr als 95 Mol-%, basierend auf der Menge des eingesetzten Alkohols R²OH, verstanden.

In einer besonders bevorzugten Ausführungsform des Verfahrens werden mehr als 50 Mol-%, vorzugsweise mehr als 60 Mol-%, bevorzugt mehr als 70 Mol-%, besonders bevorzugt mehr als 80 Mol-%, mit Vorzug mehr als 90 Mol-% und mit besonderem Vorzug mehr als 95 Mol-% und insbesondere mehr als 99 Mol-% des sich im Laufe des Verfahrens bildenden R¹OH während des Verfahrens - vorzugsweise mittels Destillation - aus dem Reaktionsgefäß entfernt.

Vorzugsweise werden während der Reaktion in regelmäßigen Abständen GC-Chromatogramme angefertigt um den Fortschritt der Reaktion zu beobachten. Mit Vorzug wird die Reaktion durch Abkühlen und/oder Zerstörung des Katalysators, beispielsweise durch Zugabe von Wasser und/oder Base, abgebrochen, nachdem in den GC-Chromatogrammen ein Restgehalt an eingesetztem Alkohol R²OH von kleiner als 5,0 Flächen-%, bevorzugt von kleiner als 2 Flächen-%, besonders bevorzugt kleiner 1,0 Flächen-% und insbesondere von kleiner 0,5 Flächen-%, bezogen auf die Gesamtfläche aller Ester im GC-Chromatogramm, festgestellt wird.

Es ist besonders bevorzugt, dass der Katalysator zerstört wird, nachdem mehr als 95 Mol-% des sich im Laufe des Verfahrens bildenden R¹OH aus dem Reaktionsgefäß entfernt wurden. Hierbei findet die Zerstörung des Katalysators vorzugsweise statt, nachdem mittels Reaktionskontrolle ein Reaktionsfortschritt von mindestens 90 % festgestellt wurde, beispielsweise indem in den Reaktionsfortschritt kontrollierenden GC-Chromatogrammen ein Restgehalt an eingesetztem R²OH von kleiner als 5,0 Flächen-%, bevorzugt von kleiner als 2,0 Flächen-% und insbesondere von kleiner 1,0 Flächen-%, bezogen auf die Gesamtfläche aller Ester im GC-Chromatogramm, festgestellt wird.

Nach Beendigung der Reaktion wird das Reaktionsgemisch in üblicher Art und Weise aufgearbeitet.

Bevorzugter Gegenstand der vorliegenden Erfindung ist ein Verfahren zur Herstellung eines Estergemisches umfassend die Ester A(COOR¹)(COOR¹)ₓ, A(COOR¹)(COOR²)ₓ, A(COOR²)(COOR¹)ₓ und A(COOR²)(COOR²)ₓ, in welchem der Ester A(COOR¹)(COOR¹)ₓ mit weniger als 1 Mol-Äquivalent, bezogen auf die Anzahl seiner Esterfunktionen, des Alkohols R²OH in Anwesenheit eines Katalysators umgesetzt und dabei zum Sieden erhitzt wird, wobei der Alkohol R¹OH einen niedrigeren Siedepunkt bei einem bestimmten Druck aufweist als der Alkohol R²OH bei demselben Druck und mehr als 90 Mol-% und insbesondere mehr als 95 Mol-% des sich im Laufe des Verfahrens bildenden R¹OH während des Verfahrens - vorzugsweise mittels Destillation - aus dem Reaktionsgefäß entfernt werden und
- A: für einen aromatischen, alicyclischen oder aliphatischen Rest steht,
- x: 1 oder 2 ist und
- R¹ und R²: unabhängig voneinander für substituierte oder nichtsubstituierte Arylreste oder lineare oder verzweigte, substituierte oder nicht substituierte Alkylreste mit 3 bis 20 Kohlenstoffatomen stehen.

Hierbei weicht vorzugsweise das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte von dem statistisch ermittelten Erwartungswert ab, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Hierbei weicht vorzugsweise für x = 2 das Molverhältnis der Ester A(COOR¹)(COOR¹)ₓ, A(COOR¹)(COOR²)ₓ, A(COOR²)(COOR¹)ₓ und A(COOR²)(COOR²)ₓ um weniger als 25 Punkte, vorzugsweise um weniger als 15 Punkte und insbesondere um weniger als 10 Punkte vom statistisch ermittelten Erwartungswert ab, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Bei der voranstehend genannten bevorzugten Ausführungsform der vorliegenden Erfindung ist es besonders bevorzugt, dass der Katalysator zerstört wird, nachdem mehr als 95 Mol-% des sich im Laufe des Verfahrens bildenden R¹OH aus dem Reaktionsgefäß entfernt wurden. Hierbei findet die Zerstörung des Katalysators vorzugsweise statt, nachdem mittels Reaktionskontrolle ein Reaktionsfortschritt von mindestens 90 % festgestellt wurde, beispielsweise indem in den Reaktionsfortschritt kontrollierenden GC-Chromatogrammen ein Restgehalt an eingesetztem R²OH von kleiner als 5,0 Flächen-%, bevorzugt von kleiner als 2,0 Flächen-% und insbesondere von kleiner 1,0 Flächen-%, bezogen auf die Gesamtfläche aller Ester im GC-Chromatogramm, festgestellt wird.

In einigen Ausführungsformen des erfindungsgemäßen Verfahrens wird ein Estergemisch hergestellt, in welchem die Ester hydrierbare Funktionalitäten wie zum Beispiel C=C-Doppelbindungen oder aromatische Reste aufweisen. Diese können - optional nach einem oder mehreren Aufarbeitungsschritten - in einem nachfolgenden Verfahrensschritt hydriert und die Ester so zu gesättigten (das heißt C-C-Mehrfachbindungs- oder Aromaten-freien) Verbindungen umgesetzt werden, welche vorzugsweise geeignet sind, als Weichmacher oder Weichmacherkomponente in Polymeren, beispielsweise PVC, eingesetzt zu werden. Bevorzugt handelt es sich in dem erfindungsgemäßen Verfahren um ein Estergemisch der Phthalsäure, der Terephthalsäure, oder der Isophthalsäure und das Estergemisch wird in einem auf nachfolgenden Verfahrensschritt hydriert.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäuredi*iso*nonylester und Cyclohexandicarbonsäurepentyl(*iso*nonyl)ester, in welchem Terephthalsäuredipentylester oder Isophthalsäuredipentylester oder Phthalsäuredipentylester mit mehr als 0,05 Mol-Äquivalent aber weniger 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird und das Estergemisch in einem nachfolgenden Verfahrensschritt einer Kernhydrierung unterzogen wird. Bevorzugt handelt es sich bei den Pentyl-Resten um *Iso*pentyl-Reste.

Ein weiterer bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäuredipropylheptylester und Cyclohexandicarbonsäurepentyl(propylheptyl)ester, in welchem Terephthalsäuredipentylester oder Isophthalsäuredipentylester oder Phthalsäuredipentylester mit mehr als 0,05 Mol-Äquivalent aber weniger 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols Propylheptanol umgesetzt und dabei zum Sieden erhitzt wird und das Estergemisch nach einer optionalen Aufarbeitung in einem nachfolgenden Verfahrensschritt einer Kernhydrierung unterzogen wird. Bevorzugt handelt es sich bei den Pentyl-Resten um *Iso*pentyl-Reste und/oder bei den Propylheptyl-Resten um 2-Propylheptyl-Reste.

Vorzugsweise sind bei den beiden obigen Ausführungsformen wiederum solche Pentyl- und *Iso*nonyl-Reste enthalten, wie weiter oben im Text definiert.

Weiterer Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredi*iso*heptylester, Cyclohexandicarbonsäuredi*iso*nonylester und Cyclohexandicarbonsäure*iso*heptyl(*iso*nonyl)ester, in welchem Terephthalsäuredi*iso*heptylester oder Isophthalsäuredi*iso*heptylester oder Phthalsäuredi*iso*heptylester mit mehr als 0,05 Mol-Äquivalent aber weniger 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt wird und das Estergemisch nach einer optionalen Aufarbeitung in einem nachfolgenden Verfahrensschritt einer Kernhydrierung unterzogen wird.

Offenbart wird auch ein Estergemisch, hergestellt nach dem beschriebenen erfindungsgemäßen Verfahren, insbesondere ein Estergemisch, welches nach einem der oben beschriebenen erfindungsgemäßen Verfahren hergestellt wurde und in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Offenbart wird zudem ein Estergemisch, welches nach einem der oben beschriebenen erfindungsgemäßen Verfahren hergestellt wurde in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 2 um weniger als 25 Punkte, vorzugsweise um weniger als 15 Punkte und insbesondere um weniger als 10 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Gegenstand der vorliegenden Erfindung ist zudem die Verwendung des erfindungsgemäßen Verfahrens zur Einstellung verarbeitungs- und/oder anwendungsrelevanter Eigenschaften eines Estergemisches durch Steuerung der Mengenverteilung der Ester im Estergemisch. Beispiele für die Beeinflussung einiger im Bereich der Weichmacher verarbeitungs- und anwendungsrelevanter Eigenschaften durch die Wahl der eingesetzten Mengen an R²OH sind oben im Text aufgeführt.

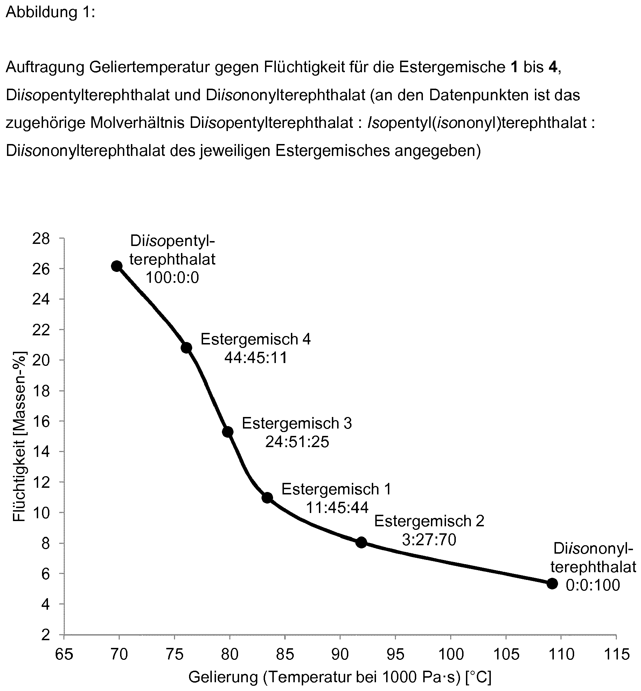

Besonders bevorzugter Gegenstand der vorliegenden Erfindung ist die Verwendung des erfindungsgemäßen Verfahrens zur Steuerung der Geliertemperatur eines Estergemisch-haltigen Plastisols und/oder der Steuerung der Flüchtigkeit eines Estergemisch-haltigen Prüfkörpers durch Steuerung der Mengenverteilung der Ester im Estergemisch. In der Abbildung 1 ist für erfindungsgemäße Estergemische mit unterschiedlicher Mengenverteilung der enthaltenen Ester die Geliertemperatur der Plastisole gegen die zugehörige Flüchtigkeit der dasselbe Estergemisch enthaltenden Folien aufgetragen. Aus dieser Abbildung wird ersichtlich, dass Produkte (Plastisole bzw. Folien) unterschiedlicher erfindungsgemäßer Gemische voneinander abweichende Geliertemperaturen und Flüchtigkeiten aufweisen und der Fachmann je nach der für die Verarbeitung und/oder Anwendung benötigten Geliertemperatur und Flüchtigkeit die geeignete Zusammensetzung eines Estergemisches auswählen kann. Selbiges gilt für andere Eigenschaften wie beispielsweise die unter anderem mittels des im experimentellen Teil beschriebenen Testes bestimmbare Verträglichkeit der Estergemische mit Polymeren, die die Weichmacher-Effizienz beschreibenden Shore-Härte, die Veränderung der Viskosität eines entsprechenden Plastisols nach Lagerung oder die Massenveränderung eines entsprechenden Plastisols bei Wasserlagerung. Das erfindungsgemäße Verfahren ermöglicht dem Fachmann nun, gezielt Estergemische mit den gewünschten Eigenschaften herzustellen, da mittels des erfindungsgemäßen Verfahrens gezielt die Mengenverteilung der Ester im Estergemisch eingestellt werden kann.

Das mittels des erfindungsgemäßen Verfahrens hergestellte Estergemisch wird vorzugsweise als Weichmacher oder als eine Komponente eines Gemisches mehrerer Polymer-weichmachender Verbindungen, welches hier ebenfalls als Weichmachergemisch bezeichnet werden soll, eingesetzt.

Offenbart wird weiterhin die Verwendung eines Estergemisches, welches gemäß dem erfindungsgemäßen Verfahren hergestellt wurde, als Weichmacher für Polymere.

Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatome(n), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und CelluloseDerivate, insbesondere Nitrocellulose (NC), Ethylcellulose (EC), Celluloseacetat (CA), Cellulose-Acetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Besonders bevorzugt ist die Verwendung eines Estergemisches, welches gemäß dem erfindungsgemäßen Verfahren hergestellt wurde, als Weichmacher für PVC.

Vorzugsweise wird das Estergemisch als Weichmacher in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, verwendet.

Offenbart wird zudem ein Mittel enthaltend ein Estergemisch, welches gemäß dem erfindungsgemäßen Verfahren hergestellt wurde, sowie ein oder mehrere Polymere aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Bezogen auf 100 Massenteile Polymer enthalten bevorzugte Mittel von 5 bis 200, bevorzugt von 10 bis 150 Massenteile an Weichmacher.

Bevorzugt ist die Verwendung des erfindungsgemäßen Estergemisches als Weichmacher für Polyvinylchlorid und dementsprechend sind Mittel, welche das erfindungsgemäße Estergemisch und PVC enthalten, besonders bevorzugt.

Bevorzugt ist das Polymer ein Suspensions-, Masse-, Mikrosuspensions- oder Emulsions-PVC.

Bevorzugte erfindungsgemäße Mittel können neben dem erfindungsgemäßen Estergemisch mindestens eine weitere Polymer-weichmachende Verbindung, also einen weiteren Weichmacher, enthalten. Eine besonders bevorzugte Ausführungsform des erfindungsgemäßen Mittels liegt vor, wenn dieses weniger als 5 Massen-% und insbesondere weniger als 0,5 Massen-% Phthalat-haltige Verbindungen enthält. Die weiteren Weichmacher werden vorzugsweise ausgewählt aus der Gruppe der Adipate, Benzoate, beispielsweise Monobenzoate oder Glycoldibenzoate, chlorierten Kohlenwasserstoffe, Citrate, Cyclohexandicarboxylate, epoxidierten Fettsäureester, epoxidierten Pflanzenöle, epoxidierten acylierten Glyceride, Furandicarboxylate, Phosphate, Phthalate (vorzugsweise in möglichst geringen Mengen), Succinate, Sulfonamide, Sulfonate, Terephthalate, Trimellitate oder oligomeren oder polymeren Ester auf Basis von Adipin-, Bernstein- oder Sebacinsäure. Besonders bevorzugt sind Alkylbenzoate, Dialkyladipate, Glycerinester, Citronensäuretrialkylester, acylierte Citronensäuretrialkylester, Trialkyltrimellitate, Glykoldibenzoate, Dialkylterephthalate, Ester der Furandicarbonsäure, Dialkanoylester von Dianhydrohexitolen (z.B. Isosorbit) und Dialkylester der 1,2-, 1,3- oder 1,4-Cyclohexandicarbonsäure.

In einer Ausführungsform enthält das erfindungsgemäße Mittel neben dem erfindungsgemäßen Estergemisch weniger als 20 Massen-%, weniger als 10 Massen-% oder keinen weiteren Weichmacher, wobei die Massen-% auf der Gesamtmasse des Mittels basieren.

Erfindungsgemäße Mittel enthalten vorzugsweise neben dem Polymer oder einer Mischung mehrerer Polymere und dem erfindungsgemäßen Estergemisch ein oder mehrere Additive aus der Gruppe der Thermostabilisatoren, Füllstoffe, Pigmente, Treibmittel, Biozide, UV- und Licht-Stabilisatoren, Co-Stabilisatoren, Antioxidantien, Viskositätsregler, Entlüfter, Haftvermittler, Gleitmittel und Färbemittel.

Die erfindungsgemäßen Mittel können in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, eingesetzt werden.

### Experimenteller Teil:

### Siedebereich der Alkohole:

Die in den Beispielen eingesetzten beziehungsweise zur Synthese der in den Beispielen eingesetzen Ester verwendeten Alkohole wiesen folgende Siedebereiche auf: *Iso*nonanol (Evonik Industries AG, Reinheit >99%): 205 bis 215 °C bei 1013 hPa; *Iso*pentanol (Gemisch *n*-Pentanol (Sigma Aldrich, Reinheit >99 %) und 2-Methylbutanol (Sigma Aldrich, Reinheit >99 %) im Molverhältnis 1:1): 129 bis 138 °C bei 1013 hPa

### Säurezahl:

Die Säurezahl wurde in Anlehnung an DIN EN ISO 2114 bestimmt.

### GC-Analysen:

Die GC- Analyse erfolgte mit folgenden Parametern:
Kapillarsäule: 30 m DB5; 0,25 mm ID; 0,25 µm Film
Trägergas: Helium
Säulenvordruck: 80 kPa
Split: ca. 23,8 ml/min
Ofentemperaturprogramm (Dauer: 51 min): 50 °C (für 1 min), Aufheizen mit 7,5 °C/min auf 350 °C (Temperatur halten für 1 min)
Injektor: 350 °C
Detektor (FID): 400 °C
Injektionsvolumen: 1,0 µl

Die Identifizierung der Komponenten im Chromatogramm der Probe erfolgte mittels einer Vergleichslösung der relevanten Ester. Im Anschluss erfolgte eine Normierung der Signale im Chromatogramm der Probe auf 100 Flächen-%. Die Stoffmengenverhältnisse wurden in hinreichender Näherung aus den Flächenverhältnissen der einzelnen Signale bestimmt.

Die Reinheit wurde über den Anteil der Produktsignale an den Gesamtflächen im Chromatogramm bestimmt.

### Herstellung der in den Beispielen 1-3 eingesetzten Terephthalate DPT und DINT

### a) Herstellung von Diisopentylterephthalat (DPT)

970 g (5 mol) Dimethylterephthalat (Sigma Aldrich, Reinheit >99%) wurden in einer Umesterungsapparatur bestehend aus einem 4 I Rührkolben mit Rührer, Tauchrohr, Thermometer und 20 cm Raschigringkolonne mit aufgesetztem Kondensator vorgelegt und in 1102 g (12,5 mol) *Iso*pentanol suspendiert. Die Apparatur wurde mindestens eine Stunde mit Stickstoff (6 l/h) über das Tauchrohr gespült. Anschließend wurden 0,25 Gew.-% Tetra-*n*-Butyltitanat (Sigma Aldrich, Reinheit >97 %) bezogen auf die Masse des Terephthalsäureesters hinzugegeben. Die Reaktionsmischung wurde langsam unter Rühren aufgeheizt. Reaktionsbeginn war bei ca. 125 °C Sumpftemperatur und ca. 62 °C Kopftemperatur. Ab diesem Zeitpunkt fiel Methanol an, das kontinuierlich über den Destillationskopf aus der Reaktion entfernt wurde. Das Produkt wurde bis zur Sumpftemperatur von 180°C weiter erhitzt. Stieg die Kopftemperatur über 65 °C, wurde kein Destillat abgenommen. Im Verlauf der Umesterung fielen ca. 320 g Methanol an (10 mol). Die Reaktionszeit betrug ca. 4 h. Ab Reaktionsbeginn bei ca.125 °C wurden stündlich Proben genommen und diese gaschromatografisch analysiert. Sobald in der GC-Analyse weniger als 0,5 Flächen-% Methyl(pentyl)terephthalat zu erkennen waren, wurde weiteres Destillat abdestilliert, indem der Ansatz langsam bei abgestellter Heizung vom Umgebungsdruck bis hin zum maximalen Vakuum (ca. 1 mbar) evakuiert wurde. Die Sumpftemperatur wurde auf 160 °C gehalten und das anfallende Destillat wurde vollständig abgenommen. Der Reaktionskolben wurde nachfolgend an eine Claisenbrücke mit Vakuumvorstoß angebaut. Außerdem wurde der Kolben mit Tauchrohr und Thermometer bestückt. Vom Kolbeninhalt wurde die Säurezahl bestimmt. Entsprechend des Ergebnisses wurde das Reaktionsmedium durch Zugabe der dreifachen stöchiometrischen Menge an Base (10 %-ige wässrige NaOH-Lösung) neutralisiert und unter Einleitung von Stickstoff (6 l/h) bei 80 °C für 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurden. Der Stickstoffstrom wurde so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt.

Nach Abkühlen wurde das Rohprodukt über einen Büchnertrichter mit Filterpapier und vorgepresstem Filterkuchen aus Filterhilfsmittel (Perlite Typ D14) mittels Vakuum in eine Saugflasche filtriert.

Es wurden 1440 g Di*iso*pentylterephthalat (DPT) mit einer Reinheit von 99,74 Flächen-% (gemäß GC-Analyse) erhalten. Das DPT wurde im nachfolgenden Beispiel 1 eingesetzt.

### b) Herstellung von Diisononylterephthalat (DINT)

776 g (4 mol) Dimethylterephthalat (Sigma Aldrich, Reinheit >99%) wurden in einer Umesterungsapparatur bestehend aus einem 4 I Rührkolben mit Rührer, Tauchrohr, Thermometer und 20 cm Raschigringkolonne mit aufgesetztem Kondensator vorgelegt und in 1440 g (10 mol) *Iso*nonanol suspendiert. Die Apparatur wurde mindestens eine Stunde mit Stickstoff (6 l/h) über das Tauchrohr gespült. Anschließend wurden 0,06 Gew.-% Tetra-*n*-Butyltitanat (Sigma Aldrich, Reinheit >97 %) bezogen auf die Masse des Terephthalsäureesters hinzugegeben. Die Reaktionsmischung wurde langsam unter Rühren aufgeheizt. Reaktionsbeginn war bei ca. 155 °C Sumpftemperatur und ca. 62 °C Kopftemperatur. Ab diesem Zeitpunkt fiel Methanol an, das kontinuierlich unter leichtem Rückfluss über den Destillationskopf aus der Reaktion entfernt wurde. Stieg die Kopftemperatur über 65 °C, wurde kein Destillat abgenommen. Das Stoffgemisch wurde bis zu einer Sumpftemperatur von 240 °C weiter erhitzt. Ab 240 °C Sumpftemperatur wurde schrittweise unter Aufrechterhaltung des Rückflusses Vakuum angelegt. Dabei fiel die Kopftemperatur langsam mit Reduzierung des Drucks. Im Verlauf der Umesterung fielen ca. 256 g Methanol an (8 mol). Die Reaktionszeit betrug ca. 4 Stunden. Ab Erreichen des Reaktionsbeginns bei ca.125 °C wurden stündlich Proben genommen und diese gaschromatografisch analysiert. Sobald in der GC-Analyse weniger als 0,5 % Methyl(*iso*nonyl)ester zu erkennen waren, wurde der Inhalt des Reaktionskolbens durch Einleiten von Stickstoff auf 80 °C gekühlt und an eine Claisenbrücke mit Vakuumteiler angebaut. Außerdem wurde der Kolben mit Tauchrohr mit aufgesetztem Tropftrichter und Thermometer bestückt. Zur Destillation des Überschussalkohols wurde der Ansatz langsam bei abgestellter Heizung vom Umgebungsdruck bis hin zum maximalen Vakuum evakuiert (ca. 1 mbar). Danach wurde die Heizung angestellt und bei ca.190 °C wurde das anfallende Destillat vollständig abgenommen. Das Rohprodukt wurde durch Einleiten von Stickstoff und durch Ausschalten der Heizung auf 80 °C abgekühlt. Vom Kolbeninhalt wurde die Säurezahl bestimmt. Entsprechend des Ergebnisses wurde das Reaktionsmedium durch langsames Zutropfen der dreifachen stöchiometrischen Menge an Base (10 %-ige wässrige NaOH-Lösung) neutralisiert und unter Einleitung von Stickstoff (6 l/h) bei 80 °C für 15 min. gerührt. Anschließend wurde schrittweise Vakuum angelegt und bis auf ca. 1 mbar evakuiert. Dann wurde der Ansatz auf 180°C aufgeheizt und nachfolgend bei konstanter Temperatur unter maximalem Vakuum der restliche verbliebene Alkohol durch eine Wasserdampfdestillation ausgetrieben. Dazu wurden 5 Gew.-% Wasser bezogen auf die Rohestermenge über das Tauchrohr zugetropft. Anschließend wurde die Heizung abgestellt und unter Vakuum und Zutropfen von Wasser abgekühlt. Bei 130 °C wurde das Wasser abgestellt. Der Ansatz wurde jetzt bis < 100°C unter Vakuum getrocknet und abgekühlt. Der Ester wurde über einen Büchnertrichter mit Filterpapier und vorgepresstem Filterkuchen aus Filterhilfsmittel (Perlite Typ D14) mittels Vakuum in eine Saugflasche gefiltert.

Es wurden 1630 g Di*iso*nonylterephthalat (DINT) mit einer Reinheit größer 99,5 Flächen-% (gemäß GC-Analyse) erhalten. Das DINT wurde im nachfolgenden Beispiel 2 eingesetzt.

### Beispiel 1 (erfindungsgemäß):

### Umesterung von Diisopentylterephthalat mit Isononanol

1697 g (5,5 mol) Di*iso*pentylterephthalat (DPT) wurden in einer Umesterungsapparatur bestehend aus einem 4 I Rührkolben mit Rührer, Tauchrohr, Thermometer und 20 cm Raschigringkolonne mit aufgesetztem Kondensator vorgelegt und in 1025 g (7,1 mol) *Iso*nonanol gelöst. Die Apparatur wurde mindestens eine Stunde mit Stickstoff (6 l/h) über das Tauchrohr gespült. Anschließend wurden 0,25 Gew.-% Tetra-*n*-Butyltitanat (Sigma Aldrich, Reinheit >97 %) bezogen auf die Masse des Terephthalsäureesters hinzugegeben. Die Reaktionsmischung wurde langsam unter Rühren aufgeheizt. Reaktionsbeginn war bei ca. 125 °C Sumpftemperatur. Das Gemisch wurde für 4 h zum Sieden erhitzt. Bei sprunghaftem Anstieg der Kopftemperatur wurde die Destillation durch Verschluss des Ablaufhahns unterbrochen und die Reaktion bis zur Einstellung einer stabilen Siedetemperatur am Rückfluss belassen. Während der Reaktion stieg die Sumpftemperatur bis auf 202 °C an. Im Verlaufe der Reaktion fielen 620 g C₅-Alkohole (7,0 mol) als Destillat an. Während der Reaktion wurden stündlich GC-Chromatogramme angefertigt. Sobald in diesen ein Restgehalt an *Iso*nonanol von kleiner als 0,5 Flächen-% bezogen auf die Gesamtfläche aller Terephthalsäureester festgestellt wurde, wurde durch Einleiten von Stickstoff und Abschalten der Heizung bis auf ca. 80 °C abgekühlt. Vom Kolbeninhalt wurde die Säurezahl bestimmt. Entsprechend des Ergebnisses wurde das Reaktionsmedium durch Zugabe der dreifachen stöchiometrischen Menge an Base (10 %-ige wässrige NaOH-Lösung) neutralisiert. Es wurde 1 Gew.-% Aktivkohle (Norit® CAP Super der Firma Norit (BET 1800 m²/g))zu dem Rohprodukt hinzugegeben und unter Einleitung von Stickstoff (6 l/h) bei 80 °C 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurden. Der Stickstoffstrom wurde so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Rohprodukt über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde eine GC-Analyse durchgeführt, an Hand derer die Reinheit (R) und die Zusammensetzung des Produkts (Di*iso*pentylterephthalat (C₅/C₅): *Iso*pentyl(*iso*nonyl)terephthalat (C₅/C₉): Di*iso*nonylterephthalat (C₉/C₉)) analysiert wurde.

Es wurden 2102 g aufgearbeitetes Estergemisch **1** erhalten.

**Tabelle 2: gemessene und berechnete Werte zum Beispiel 1**

| | |
|---|---|
| Stoffmenge Di*iso*pentylterephthalat | 5,5 mol |
| → Stoffmenge R¹-Äquivalente (C₅) | 11,0 mol |
| Stoffmenge *Iso*nonanol | |
| → Stoffmenge R²-Äquivalente (C₉) | 7,1 mol |
| Eingesetztes Mengenverhältnis R¹-Äquivalente zu R²-Äquivalente (C₅ : C₉) | 11,0 : 7,1 entsprechend 2,0 : 1,3 |
| Statistische Erwartungswerte für vollständigen Einbau von C₉: (C₅/C₅) : (C₅/C₉) : (C₉/C₉) | 12 : 46 : 42 (erwarteter C₅-Anteil: 35 %) |
| Zusammensetzung gemäß GC (C₅/C₅) : (C₅/C₉) : (C₉/C₉) | **11 : 45 : 44** (C₅-Anteil: 34 %) Abweichung: \|12-11\| + \|46-45\| + \|42-44\| = 4 |
| Reinheit | > 99,9 % |

### Beispiel 2 (erfindungsgemäß):

### Umesterung von Diisopentylterephthalat mit Isononanol (weitere Mengenverhältnisse R¹:R²)

Durch Umsetzung von Di*iso*pentylterephthalat mit *Iso*nonanol gemäß der im Beispiel 1 erläuterten Vorschrift können die erfindungsgemäßen Estergemische **2** bis **4** hergestellt werden:

**Tabelle 3: Herstellung weiterer erfindungsgemäßer Estergemische**

| Estergemisch | Eingesetztes Mengenverhältnis R¹-Äquivalente zu R²-Äquivalente (C₅ : C₉) | Zusammensetzung gemäß GC (C₅/C₅) : (C₅/C₉) : (C₉/C₉) |
|---|---|---|
| **2** | 2,0 : 1,7 | **3 : 27 : 70** |
| **3** | 2,0 : 1,0 | **24 : 51 : 25** |
| **4** | 2,0 : 0,7 | **44 : 45 : 11** |

### Beispiel 3 (Vergleichsbeispiel):

### Umesterung von Diisononylterephthalat mit Isopentanol

Eine Umesterungsapparatur bestehend aus Rührkolben mit Rührer, Tauchrohr, Thermometer und 20 cm Raschigringkolonne mit aufgesetztem Kondensator wurde mit 220 g (2,5 mol) *Iso*pentanol und 836 g (2,0 mol) Di*iso*nonylterephthalat (DINT) befüllt, bevor die Apparatur mindestens eine Stunde mit 6 l N₂/h über das Tauchrohr gespült wurde. Anschließend wurden 0,25 Gew.-% Tetra-*n*-Butyltitanat (Sigma Aldrich, Reinheit >97 %) bezogen auf die Masse des Terephthalsäureesters hinzugegeben. Das Gemisch wurde anschließend für 6 h zum Sieden erhitzt. Während der Reaktion stieg die Sumpftemperatur von 170 °C auf 193 °C an. Im Verlaufe der Reaktion wurde kein Destillat abgenommen. Nach 6 Stunden wurde schrittweise Vakuum (ca. 1 mbar) angelegt und bis zu einer Sumpftemperatur von 200 °C erhitzt, wobei 202 g Destillat abdestilliert wurden, bevor der Kolbeninhalt bei ausgeschalteter Heizung, durch Einleiten von Stickstoff bis auf ca. 80 °C abgekühlt wurde. Der Reaktionskolben wurde nachfolgend an eine Claisenbrücke mit Vakuumvorstoß angebaut. Außerdem wurde der Kolben mit Tauchrohr und Thermometer bestückt. Vom Kolbeninhalt wurde die Säurezahl bestimmt. Entsprechend des Ergebnisses wurde das Reaktionsmedium durch Zugabe der dreifachen stöchiometrischen Menge an Base (10 %-ige wässrige NaOH-Lösung) neutralisiert und unter Einleitung von Stickstoff (6 l/h) bei 80 °C für 15 min. gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurden. Der Stickstoffstrom wurde so eingestellt, dass der Druck nicht über 20 mbar steigt. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Produkt über einen Büchnertrichter mit Filterpapier und vorgepresstem Filterkuchen aus Filterhilfsmittel (Perlite Typ D14) mittels Vakuum in eine Saugflasche filtriert. An dem Filtrat wird eine GC-Analyse durchgeführt, an Hand derer die Reinheit (R) und die Zusammensetzung des Produkts (Di*iso*pentylterephthalat (C₅/C₅): *Iso*pentyl(*iso*nonyl)terephthalat (C₅/C₉): Di*iso*nonylterephthalat (C₉/C₉)) analysiert wurde.

Es wurden 846 g aufgearbeitetes Estergemisch **5** erhalten.

**Tabelle 4: gemessene und berechnete Werte zum Beispiel 2**

| | |
|---|---|
| Stoffmenge Di*iso*nonylterephthalat | 2,0 mol |
| → Stoffmenge R²-Äquivalente (C₉) | 4,0 mol |
| Stoffmenge *Iso*pentanol | |
| → Stoffmenge R¹-Äquivalente (C₅) | 2,5 mol |
| Eingesetztes Mengenverhältnis R²-Äquivalente zu R¹-Äquivalente (C₉ : C₅) | 4,0 : 2,5 entsprechend 2,0 : 1,25 |
| Statistische Erwartungswerte für vollständigen Einbau von C₅: (C₅/C₅) : (C₅/C₉) : (C₉/C₉) | 42 : 46 : 12 (erwarteter C₅-Anteil: 65 %) |
| Statistische Erwartungswerte für 0 % Einbau von C₅: (C₅/C₅) : (C₅/C₉) : (C₉/C₉) | 0 : 0 : 100 (erwarteter C₅-Anteil: 0 %) |
| Zusammensetzung gemäß GC (C₅/C₅) : (C₅/C₉) : (C₉/C₉) | **1 : 21 : 78** (C₅-Anteil: 11 %) Abweichung: \|42-1\| + \|46-21\| + \|12-78\| = 132 bzw. \|0-1\| + \|0-21\| + \|100-78\| = 44 |
| R (Reinheit) | > 99,9% |

### Beispiel 4:

### Herstellung von Plastisolen der erfindungsgemäßen Estergemische

Es wurden PVC-Plastisole hergestellt, wie sie beispielsweise zur Fertigung von Deckstrichfilmen für Fußbodenbeläge verwendet werden. Die Angaben in den Plastisolrezepturen sind jeweils in Massenanteilen. Die Rezepturen der Polymerzusammensetzungen sind in Tabelle 5 aufgelistet.

**Tabelle 5: Plastisolrezeptur**

| | **P1** | **P2** | **P3** | **P4** |
|---|---|---|---|---|
| PVC (Vestolit B 7021 - Ultra; Fa. Vestolit) | 100 | 100 | 100 | 100 |
| Estergemisch **1** (11:45:44) | 50 | | | |
| Estergemisch **2** (3:27:70) | | 50 | | |
| Estergemisch **3** (24:51:25) | | | 50 | |
| Estergemisch **4** (44:45:11) | | | | 50 |
| epoxidiertes Sojabohnenöl als Co-Stabilisator (Drapex 39, Fa. Galata) | 3 | 3 | 3 | 3 |
| Thermostabilisator auf Ca/Zn-Basis (Mark CZ 149, Fa. Galata) | 2 | 2 | 2 | 2 |

| | | | | |
|---|---|---|---|---|
| Angaben in pnr (phr = paris per hundred parts resin); P: Plastisol | | | | |

Die Estergemische wurden vor der Zugabe auf 25 °C temperiert. Zuerst wurden die flüssigen und dann die pulverförmigen Bestandteile in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sichergestellt, dass die Homogenisierung des Plastisols bei einem definierten Energieeintrag erreicht wurde. Danach wurde das Plastisol sofort für weitere Untersuchungen in einem Klimaschrank auf 25,0 °C temperiert.

### Beispiel 5:

### Geliertemperatur der Plastisole 1 bis 4 (P1 - P4)

Die Untersuchung des Gelierverhaltens der Plastisole wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

### Folgende Parameter wurden eingestellt:

| Modus: | Temperatur-Gradient | |
|---|---|---|
| | Start-Temperatur: | 25 °C |
| | End-Temperatur: | 180 °C |
| | Heiz/Kühlrate: | 5 °C / min |
| | Oszillations-Frequenz: | 4 - 0,1 Hz Rampe logarithmisch |
| | Kreisfrequenz Omega: | 10 1 / s |
| | Anzahl Messpunkte: | 63 |
| | Messpunktdauer: | 0,5 min |
| | Automatische Spaltnachführung F : | 0 N |
| | Konstante Messpunktdauer | |
| | Spaltweite | 0,5 mm |

### Durchführung der Messung:

Auf die untere Messsystemplatte wurden mit dem Spatel einige Gramm des zu messenden Plastisols luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als 6 mm rundum). Anschließend wurde die Temperierhaube über der Probe positioniert und die Messung gestartet. Bestimmt wurde die sogenannte komplexe Viskosität des Plastisols nach 24 h (Lagerung des Plastisols bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Plastisolviskosität von 1000 Pa·s verwendet.

**Tabelle 6: Gelierung der Plastisole 1 bis 4 nach 24 h, Temperatur in °C bei Erreichen einer Plastisolviskosität von 10³ Pa·s (kurz: Geliertemperatur)**

| | Geliertemperatur [°C] |
|---|---|
| DINT (0:0:100) | 109 |
| **P1** (11:45:44) | 83 |
| **P2** (3:27:70) | 92 |
| **P3** (24:51:25) | 80 |
| **P4** (44:45:11) | 76 |
| DPT (100:0:0) | 70 |

| | |
|---|---|
| P: Plastisol | |

In der Abbildung 1 ist für die erfindungsgemäße Estergemische **1** bis **4** die Geliertemperatur des Plastisols aus der Tabelle 6 gegen die zugehörige Flüchtigkeit der dasselbe Estergemisch enthaltenen Folie (aus Beispiel 7, Tabelle 7) aufgetragen.

### Beispiel 6:

### Herstellung von Folien der erfindungsgemäßen Estergemische

Die im Beispiel 4 hergestellten Plastisole wurden jeweils zu 1 mm dicken Folien verarbeitet.

Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 3 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

Aus den Folien wurden 3 Kreise von 10 cm² pro zu prüfender Rezeptur ausgestanzt. Zusätzlich wurden die Kreise mit der Schere radial angeschnitten (2 Schnitte a 5 mm). Die Kreise wurden für eine halbe Stunde im Exsikkator (gefüllt mit KC- Trockenperlen orange) klimatisiert und anschließend ausgewogen.

### Beispiel 7:

### Massenverlust bei Aktivkohlelagerung bei den Folien 1 - 4 (F1 - F4)

Weißblechdosen (1 L, hohe Form) wurden, damit ein Druckaustausch stattfinden konnte, im Deckel mit einem Loch versehen. Der Boden der Weißblechdosen wurde mit 120 mL Aktivkohle bedeckt. Die in diesem Test eingesetzte Aktivkohle (Nr. 774408 der Fa. Roth) wurde vorher in einer Abdampfschale für 6 Stunden im Trockenschrank bei 100 +/-1 °C getrocknet und nach kurzer Abkühlung eingesetzt. Auf die Aktivkohle wurde der erste Probenkreis mittig platziert. Weitere 120 mL Aktivkohle wurden auf den Probenkreis gegeben. Insgesamt wurden die Weißblechdosen mit 480 mL Aktivkohle und 3 Probenkreisen schichtweise befüllt. Der Deckel der Weißblechdosen wurde ohne Druck auf die Dosen aufgelegt.

Die befüllten Weißblechdosen wurden bei 100 +/-1 °C für 3 Tage im Temperierschrank gelagert. Nach der Lagerung wurde die Aktivkohle von den Kreisen mittels Analysenpinsel entfernt, die Kreise zum Abkühlen 30 Minuten in einem Exsikkator gelagert und anschließend ausgewogen.

Nach der Auswaage wurden die Probenkreise wieder mit Aktivkohle zurück in die Weißblechdosen geschichtet. Hierzu wurde darauf geachtet, dass die Probenkreise wieder derselben Aktivkohle und derselben Dose zugeordnet wurden. Die Dosen wurden erneut im Temperierschrank platziert. Nach insgesamt 7 Tagen wurden die Proben dann, wie bereits beschrieben, nochmals ausgewogen.

Es wurde die prozentuale Massenveränderung von jedem Probenkreis berechnet und der Mittelwert über die 3 Kreise je Rezeptur berechnet.

**Tabelle 7: Massenverlust bei Aktivkohlelagerung in Massen-% (Flüchtigkeit)**

| | Flüchtigkeit [Massen-%] | |
|---|---|---|
| | 3 Tage | 7 Tage |
| DINT | 4,0 | 5,4 |
| **F1** (11:45:44) | 6,2 | 10,8 |
| **F2** (3:27:70) | 4,9 | 8,2 |
| **F3** (24:51:25) | 9,3 | 15,3 |
| **F4** (44:45:11) | 13,9 | 20,8 |
| DPT | 20,5 | 26,2 |

| | | |
|---|---|---|
| F: Folie | | |

In der Abbildung 1 ist für die erfindungsgemäße Estergemische **1** bis **4** die Geliertemperatur des Plastisols aus der Tabelle 6 des Beispiels 5 gegen die zugehörige Flüchtigkeit der dasselbe Estergemische enthaltenen Folie (aus Tabelle 7) aufgetragen.

## Patentansprüche

1. Verfahren zur Herstellung eines Estergemisches umfassend die Ester A(COOR¹)(COOR¹)ₓ, A(COOR¹)(COOR²)ₓ, A(COOR²)(COOR¹)ₓ und A(COOR²)(COOR²)ₓ, in welchem der Ester A(COOR¹)(COOR¹)ₓ mit weniger als 1 Mol-Äquivalent, bezogen auf die Anzahl seiner Esterfunktionen, des Alkohols R²OH in Anwesenheit eines Katalysators umgesetzt und dabei zum Sieden erhitzt wird, wobei der Alkohol R¹OH einen niedrigeren Siedepunkt bei einem bestimmten Druck aufweist als der Alkohol R²OH bei demselben Druck und mehr als 90 Mol-% des sich im Laufe des Verfahrens bildenden R¹OH während des Verfahrens aus dem Reaktionsgefäß entfernt werden bevor der Katalysator zerstört wird und
A für einen aromatischen, alicyclischen oder aliphatischen Rest steht,
x 1 oder 2 ist und
R¹ und R² unabhängig voneinander für substituierte oder nichtsubstituierte Arylreste oder lineare oder verzweigte, substituierte oder nicht substituierte Alkylreste mit 3 bis 20 Kohlenstoffatomen stehen.

2. Verfahren gemäß Anspruch 1, **dadurch gekennzeichnet, dass** es sich um ein Estergemisch der Phthalsäure, der Terephthalsäure, der Isophthalsäure, der 1,2-, 1,3- oder 1,4- Cyclohexandicarbonsäure, der Adipinsäure, der Sebacinsäure, der Maleinsäure, der Bernsteinsäure, der Furandicarbonsäure oder der Citronensäure handelt.

3. Verfahren gemäß einem der Ansprüche 1 oder 2, **dadurch gekennzeichnet, dass** R¹ und R² unabhängig voneinander ausgewählt sind aus Alkylresten, welche 3 bis 20 Kohlenstoffatome enthalten.

4. Verfahren gemäß einem der Ansprüche 1 bis 3, **dadurch gekennzeichnet, dass** R¹ und R² unabhängig voneinander ausgewählt sind aus Propyl-, Butyl-, *tert*-Butyl-, *Iso*butyl-, 2-Methylbutyl-, 3-Methylbutyl-, *n*-Pentyl-, *Iso*pentyl-, Hexyl-, Heptyl-, *Iso*heptyl-, Octyl-, *Iso*octyl-, 2-Ethylhexyl-, Nonyl-, *n*-Nonyl-, *Iso*nonyl-, Decyl-, *Iso*decyl-, 2-Propyl-heptyl-, Undecyl- und Tridecyl- Resten.

5. Verfahren gemäß einem der Ansprüche 1 bis 4, **dadurch gekennzeichnet, dass** die Siedepunkte der Alkohole R¹OH und R²OH sich um mindestens 10 °C unterscheiden.

6. Verfahren gemäß einem der Ansprüche 1 bis 5, **dadurch gekennzeichnet, dass** A(COOR¹)(COOR¹)ₓ mit weniger als 0,98 Mol-Äquivalent, bezogen auf die Anzahl seiner Esterfunktionen, des Alkohols R²OH umgesetzt wird.

7. Verfahren gemäß einem der Ansprüche 1 bis 6, **dadurch gekennzeichnet, dass** A(COOR¹)(COOR¹)ₓ mit mehr als 0,05 Mol-Äquivalent, bezogen auf die Anzahl seiner Esterfunktionen, des Alkohols R²OH umgesetzt wird.

8. Verfahren gemäß einem der Ansprüche 1 bis 7, **dadurch gekennzeichnet, dass** es ein Estergemisch bereitstellt, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte vom statistisch ermittelten Erwartungswert abweicht, welcher sich ergibt, wenn man von einem vollständigen Einbau der Alkoholreste OR² ausgeht, wobei dieser Punkte-Wert der Summe der Beträge aller Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

9. Verfahren gemäß einem der Ansprüche 1 bis 7, **dadurch gekennzeichnet, dass** es ein Estergemisch bereitstellt, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 2 um weniger als 25 Punkte vom statistisch ermittelten Erwartungswert abweicht, welcher sich ergibt, wenn man von einem vollständigen Einbau der Alkoholreste OR² ausgeht, wobei dieser Punkte-Wert der Summe der Beträge aller Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

10. Verfahren gemäß einem der Ansprüche 1 bis 9, **dadurch gekennzeichnet, dass** es sich um ein Estergemisch der Phthalsäure, der Terephthalsäure oder der Isophthalsäure handelt und das Estergemisch nach einer optionalen Aufarbeitung in einem nachfolgenden Verfahrensschritt hydriert wird.

11. Verwendung eines Verfahrens gemäß einem der Ansprüche 1 bis 10 zur Einstellung verarbeitungs- und/oder anwendungsrelevanter Eigenschaften eines Estergemisches durch Steuerung der Mengenverteilung der Ester im Estergemisch.

## Claims

1. Process for the preparation of an ester mixture comprising the esters A(COOR¹)(COOR¹)ₓ, A(COOR¹)(COOR²)ₓ, A(COOR²)(COOR¹)ₓ and A(COOR²)(COOR²)ₓ, in which the ester A(COOR¹)(COOR¹)ₓ is reacted with less than 1 molar equivalent, based on the number of ester functions of said ester A(COOR¹)(COOR¹)ₓ, of the alcohol R²OH in the presence of a catalyst and heated to boiling at the same time, wherein the alcohol R¹OH has a lower boiling point at a given pressure than the alcohol R²OH at the same pressure, and more than 90 mol% of the R¹OH forming in the course of the process are removed from the reaction vessel during the process before the catalyst is destroyed, and
A is an aromatic, alicyclic or aliphatic radical,
x is 1 or 2, and
R¹ and R², independently of one another, are substituted or unsubstituted aryl radicals or linear or branched, substituted or unsubstituted alkyl radicals having 3 to 20 carbon atoms.

2. Process according to Claim 1, **characterized in that** the ester mixture is an ester mixture of phthalic acid, terephthalic acid, isophthalic acid, 1,2-cyclohexanedicarboxylic acid, 1,3-cyclohexanedicarboxylic acid or 1,4-cyclohexanedicarboxylic acid, adipic acid, sebacic acid, maleic acid, succinic acid, furandicarboxylic acid or citric acid.

3. Process according to either of Claims 1 and 2, **characterized in that** R¹ and R², independently of one another, are selected from alkyl radicals containing 3 to 20 carbon atoms.

4. Process according to one of Claims 1 to 3, **characterized in that** R¹ and R², independently of one another, are selected from propyl, butyl, tert-butyl, isobutyl, 2-methylbutyl, 3-methylbutyl, n-pentyl, isopentyl, hexyl, heptyl, isoheptyl, octyl, isooctyl, 2-ethylhexyl, nonyl, n-nonyl, isononyl, decyl, isodecyl, 2-propylheptyl, undecyl and tridecyl radicals.

5. Process according to one of Claims 1 to 4, **characterized in that** the boiling points of the alcohols R¹OH and R²OH differ by at least 10°C.

6. Process according to one of Claims 1 to 5, **characterized in that** A(COOR¹)(COOR¹)ₓ is reacted with less than 0.98 molar equivalent, based on the number of ester functions of said ester A(COOR¹)(COOR¹)ₓ, of the alcohol R²OH.

7. Process according to one of Claims 1 to 6, **characterized in that** A(COOR¹)(COOR¹)ₓ is reacted with more than 0.05 molar equivalent, based on the number of ester functions of said ester A(COOR¹)(COOR¹)ₓ, of the alcohol R²OH.

8. Process according to one of Claims 1 to 7, **characterized in that** it provides an ester mixture in which the molar ratio of the esters A(COOR¹)(COOR¹)ₓ, A(COOR¹)(COOR²)ₓ, A(COOR²)(COOR¹)ₓ and A(COOR²)(COOR²)ₓ, where x=1, deviates by less than 15 points from the statistically determined expected value that results from an assumption of complete incorporation of the alcohol radicals OR², this points value corresponding to the sum of the amounts of all the differences between statistical expected value and actual molar fraction of each individual ester in the ester mixture, when the sum of the molar fractions of the abovementioned esters in the ester mixture adds up to 100.

9. Process according to one of Claims 1 to 7, **characterized in that** it provides an ester mixture in which the molar ratio of the esters A(COOR¹)(COOR¹)ₓ, A(COOR¹)(COOR²)ₓ, A(COOR²)(COOR¹)ₓ and A(COOR²)(COOR²)ₓ, where x=2, deviates by less than 25 points from the statistically determined expected value that results from an assumption of complete incorporation of the alcohol radicals OR², this points value corresponding to the sum of the amounts of all the differences between statistical expected value and actual molar fraction of each individual ester in the ester mixture, when the sum of the molar fractions of the abovementioned esters in the ester mixture adds up to 100.

10. Process according to one of Claims 1 to 9, **characterized in that** the ester mixture is an ester mixture of phthalic acid, terephthalic acid or isophthalic acid, and the ester mixture is hydrogenated after an optional work-up in a subsequent process step.

11. Use of a process according to one of Claims 1 to 10 for adjusting processing-relevant and/or application-relevant properties of an ester mixture by controlling the quantitative distribution of the esters in the ester mixture.

## Revendications

1. Procédé de fabrication d'un mélange d'esters comprenant les esters A(COOR¹)(COOR¹)ₓ, A(COOR¹)(COOR²)ₓ, A(COOR²)(COOR¹)ₓ et A(COOR²)(COOR²)ₓ, dans lequel l'ester A(COOR¹)(COOR¹)ₓ est mis en réaction avec moins de 1 équivalent molaire, par rapport au nombre de ses fonctions ester, de l'alcool R²OH en présence d'un catalyseur, et porté à ébullition, l'alcool R¹OH présentant un point d'ébullition à une pression déterminée plus bas que l'alcool R²OH à la même pression, et plus de 90 % en moles du R¹OH qui se forme au cours du procédé étant éliminé du récipient de réaction pendant le procédé avant que le catalyseur ne soit détruit, et
A représentant un radical aromatique, alicyclique ou aliphatique,
x représentant 1 ou 2, et
R¹ et R² représentant indépendamment l'un de l'autre des radicaux aryle substitués ou non substitués, ou des radicaux alkyle linéaires ou ramifiés, substitués ou non substitués, de 3 à 20 atomes de carbone.

2. Procédé selon la revendication 1, **caractérisé en ce qu'**il s'agit d'un mélange d'esters de l'acide phtalique, de l'acide téréphtalique, de l'acide isophtalique, de l'acide 1,2-, 1,3- ou 1,4-cyclohexanedicarboxylique, de l'acide adipique, de l'acide sébacique, de l'acide maléique, de l'acide succinique, de l'acide furanedicarboxylique ou de l'acide citrique.

3. Procédé selon l'une quelconque des revendications 1 ou 2, **caractérisé en ce que** R¹ et R² sont choisis indépendamment l'un de l'autre parmi les radicaux alkyle qui contiennent 3 à 20 atomes de carbone.

4. Procédé selon l'une quelconque des revendications 1 à 3, **caractérisé en ce que** R¹ et R² sont choisis indépendamment l'un de l'autre parmi les radicaux propyle, butyle, *tert*-butyle, *iso*butyle, 2-méthylbutyle, 3-méthylbutyle, *n*-pentyle, *iso*pentyle, hexyle, heptyle, *iso*heptyle, octyle, *iso*octyle, 2-éthylhexyle, nonyle, n-nonyle, *iso*nonyle, décyle, *iso*décyle, 2-propyl-heptyle, undécyle et tridécyle.

5. Procédé selon l'une quelconque des revendications 1 à 4, **caractérisé en ce que** les points d'ébullition des alcools R¹OH et R²OH diffèrent d'au moins 10 °C.

6. Procédé selon l'une quelconque des revendications 1 à 5, **caractérisé en ce qu'**A(COOR¹)(COOR¹)ₓ est mis en réaction avec moins de 0,98 équivalent molaire, par rapport au nombre de ses fonctions ester, de l'alcool R²OH.

7. Procédé selon l'une quelconque des revendications 1 à 6, **caractérisé en ce qu'**A(COOR¹)(COOR¹)ₓ est mis en réaction avec plus de 0,05 équivalent molaire, par rapport au nombre de ses fonctions ester, de l'alcool R²OH.

8. Procédé selon l'une quelconque des revendications 1 à 7, **caractérisé en ce qu'**il met à disposition un mélange d'esters dans lequel le rapport molaire entre les esters A(COOR¹)(COOR¹)ₓ, A(COOR¹)(COOR²)ₓ, A(COOR²)(COOR¹)ₓ et A(COOR²)(COOR²)ₓ pour x = 1 diffère de moins de 15 points de la valeur attendue calculée statistiquement qui est obtenue en supposant une incorporation totale des radicaux alcool OR², cette valeur de point correspondant à la somme des montants de toutes les différences entre la valeur attendue statistique et la proportion molaire réelle de chaque ester individuel dans le mélange d'esters lorsque la somme des proportions molaires des esters mentionnés précédemment dans le mélange d'esters est de 100.

9. Procédé selon l'une quelconque des revendications 1 à 7, **caractérisé en ce qu'**il met à disposition un mélange d'esters dans lequel le rapport molaire entre les esters A(COOR¹)(COOR¹)ₓ, A(COOR¹)(COOR²)ₓ, A(COOR²)(COOR¹)ₓ et A(COOR²)(COOR²)ₓ pour x = 2 diffère de moins de 25 points de la valeur attendue calculée statistiquement qui est obtenue en supposant une incorporation totale des radicaux alcool OR², cette valeur de point correspondant à la somme des montants de toutes les différences entre la valeur attendue statistique et la proportion molaire réelle de chaque ester individuel dans le mélange d'esters lorsque la somme des proportions molaires des esters mentionnés précédemment dans le mélange d'esters est de 100.

10. Procédé selon l'une quelconque des revendications 1 à 9, **caractérisé en ce qu'**il s'agit d'un mélange d'esters de l'acide phtalique, de l'acide téréphtalique ou de l'acide isophtalique, et le mélange d'esters est hydrogéné après un traitement optionnel dans une étape de procédé ultérieure.

11. Utilisation d'un procédé selon l'une quelconque des revendications 1 à 10 pour l'ajustement des propriétés relatives à l'usinage et/ou à l'utilisation d'un mélange d'esters par régulation de la répartition des quantités des esters dans le mélange d'esters.
